# FASCICULE DE BREVET EUROPEEN

(11) **EP 0 854 880 B1**
(45) Date de publication et mention de la délivrance du brevet: **06.05.2004**
(21) Numéro de dépôt: 96931098.6
(22) Date de dépôt: 10.09.1996
(51) Int. Cl.: C07H 17/08

(54) **DERIVES DE LA 5-0-DESOSAMINYL 6-0-METHYL ERYTHRONOLIDE A, LEUR PROCEDE DE PREPARATION ET LEUR APPLICATION A LA PREPARATION DE PRODUITS BIOLOGIQUEMENT ACTIFS**
5-0-DESOSAMINYL 6-0-METHYL ERYTHRONOLID A, IHRE VERFAHREN ZUR HERSTELLUNG UND IHRE VERWENDUNG ZUR HERSTELLUNG EIN ARZNEIMITTEL
5-O-DESOSAMINYL 6-O-METHYL ERYTHRONOLIDE DERIVATIVES A, METHOD FOR PREPARING SAME AND APPLICATION THEREOF FOR PREPARING BIOLOGICALLY ACTIVE PRODUCTS

(30) Priorité: 11.09.1995 FR 9510601
(43) Date de publication de la demande: 29.07.1998
(73) Titulaire: Aventis Pharma S.A., 92160 Antony (FR)
(72) Inventeur: BONNET, Alain, F-77100 Meaux (FR); DELTHIL, Michel, F-93130 Noisy-le-Sec (FR); MAZURIE, Alain, F-93410 Vaujours (FR)
(86) Numéro de dépôt international: PCT/FR1996/001384
(87) Numéro de publication internationale: WO 1997/010251

(56) Documents cités:
- EP-A- 0 272 110
- EP-A- 0 619 319
- EP-A- 0 619 320

## Description

La présente invention concerne de nouveaux dérivés de la 5-O-désosaminyl 6-O-méthyl érythronolide A, leur procédé de préparation et leur application à la préparation de produits biologiquement actifs.

On connaissait des dérivés 3-0-acétyle de la 5-0-désosaminyl-6-0-méthyl érythronolide A doués de propriétés antibiotiques.

L'invention a pour objet les composés de formule (I) : 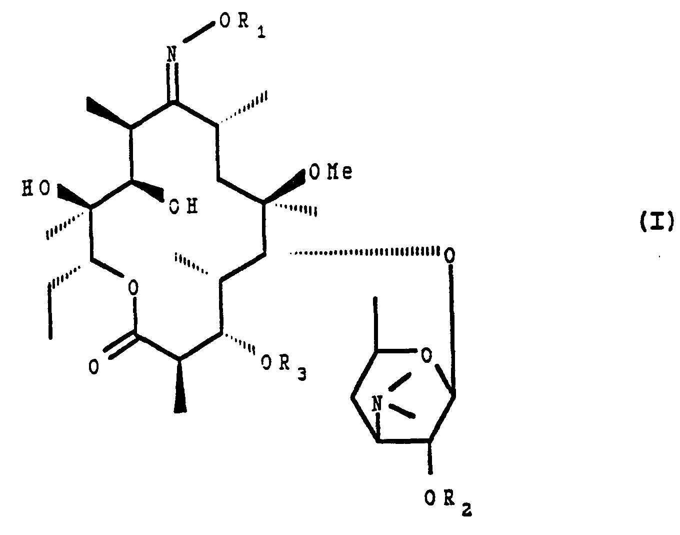 dans lesquels :
ou bien R₁ représente un radical alkyle renfermant jusqu'à 8 atomes de carbone substitué par un ou plusieurs radicaux alkyle renfermant jusqu'à 8 atomes de carbone, ou par un ou plusieurs radicaux aryle renfermant jusqu'à 14 atomes de carbone,
ou bien R₁ représente un radical aryle renfermant jusqu'à 14 atomes de carbone, éventuellement substitué par un ou plusieurs radicaux alkyle, alkényle ou alkynyle renfermant jusqu'à 8 atomes de carbone, alkoxy ou alkylthio renfermant jusqu'à 8 atomes de carbone, nitro, CF₃ ou par un ou plusieurs atomes d'halogène, 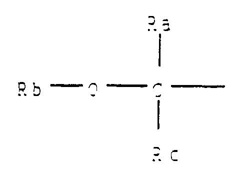 dans lequel Ra représente un radical alkyle ou alkoxy renfermant jusqu'à 8 atomes de carbone,
Rb représente un radical alkyle renfermant jusqu'à 8 atomes de carbone, éventuellement substitué par un hétéroatome,
Rc représente un atome d'hydrogène ou un radical alkyle renfermant jusqu'à 8 atomes de carbone,
R₂ et R₃, identiques ou différents, représentent un radical trialkylsilyle dans lequel le radical alkyle renferme jusqu'à 8 atomes de carbone.

Dans la définition des composés de l'invention :
- le radical alkyle, alkényle ou alkynyle est de préférence un radical méthyle, éthyle, propyle, isopropyle, n-butyle, isobutyle, terbutyle, décyle ou dodécyle, vinyle, allyle, éthynyle, propynyle, cyclobutyle, cyclopentyle ou cyclohexyle,
- l'halogène est de préférence le fluor ou le chlore, ou le brome,
- le radical aryle est de préférence le radical phényle, ou un radical naphtyle,
- le radical aralkyle est de préférence un radical (C₆H₅)-(CH₂)ₐ, a étant un nombre entier compris entre 1 et 6, par exemple le nombre 1, 2, 3 ou 4 ; le radical aralkyle peut être par exemple, un radical benzyle éventuellement substitué ou un radical trityle,
- le radical alkyloxy est de préférence un radical méthoxy, éthoxy, propyloxy isopropyloxy, n-butyloxy, isobutyloxy, tert-butyloxy, n-pentyloxy, isopentyloxy, sec-pentyloxy, tert-pentyloxy, néopentyloxy, n-hexyloxy, sec-hexyloxy, tert-hexyloxy,
- le radical alkylthio correspondant peut être utilisé en reprenant les mêmes valeurs et en remplaçant l'atome d'oxygène par un atome de soufre, exemple : méthylthio, éthylthio. De plus, l'atome de soufre peut être oxydé, exemple : méthylsulfinyle, méthylsulfonyle.

Les composés de l'invention peuvent être utilisés pour la préparation des composés de formule (VI) : 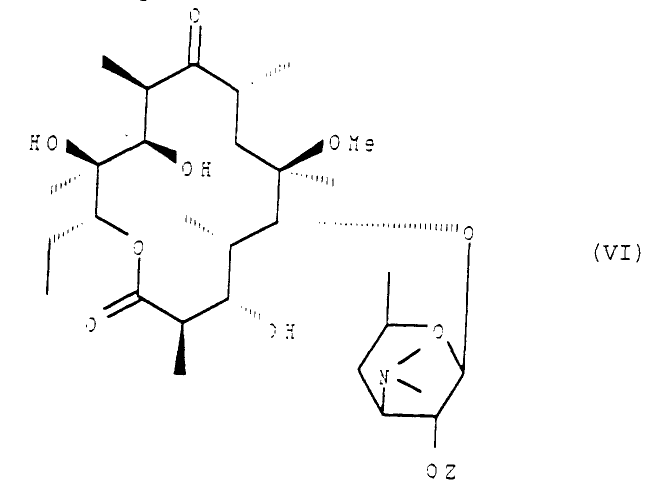 dans lesquels Z représente un atome d'hydrogène ou un groupement protecteur comme le reste d'un acide carboxylique renfermant jusqu'à 8 atomes de carbone, un radical trialkylsilyle ou terbutyle. Les composés de formule (VI) sont décrits et revendiqués dans la demande de brevet européen 0 487 411, en tant qu'intermédiaires utiles notamment pour la préparation de produits antibiotiques.

L'invention a plus particulièrement pour objet, les composés de formule (I) dans lesquels R₁ représente un radical 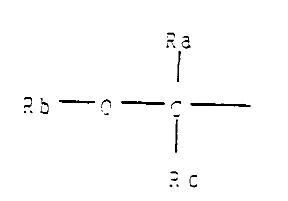 dans lequel Ra, Rb et Rc conservent la même signification que précédemment et notamment ceux dans lesquels Ra, Rb et Rc représentent un radical méthyle ainsi que les composés de formule (I) dans lesquels R₂ et R₃ représentent tous les deux un un radical triméthylsilyle.

L'invention a plus particulièrement pour objet le composé de formule (I) dont la préparation est donnée ci-après dans la partie expérimentale.

L'invention a également pour objet un procédé de préparation des composés de formule (I) tels que définis précédemment, caractérisé en ce que l'on soumet le composé de formule (II) : 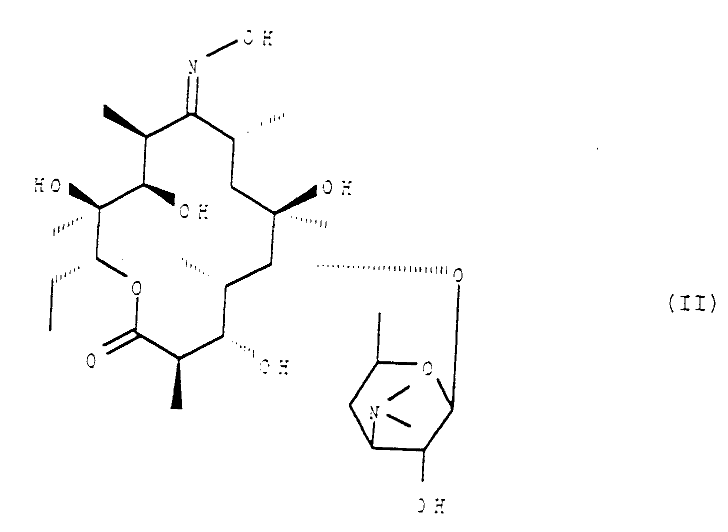 à l'action d'un agent de blocage de l'oxime en 9, pour obtenir un composé de formule (III) : 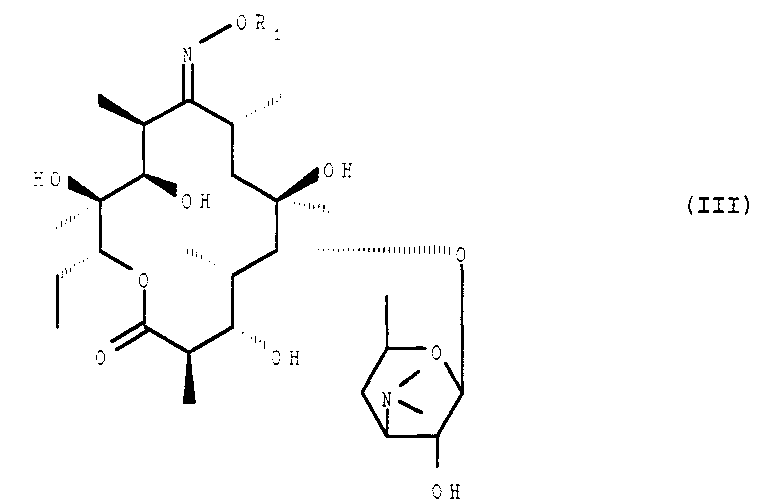 dans lequel R₁ conserve sa signification précédente, que l'on soumet à l'action d'un agent de blocage de l'hydroxyle en 3 et/ou en 2' pour obtenir le composé de formule (IV) : 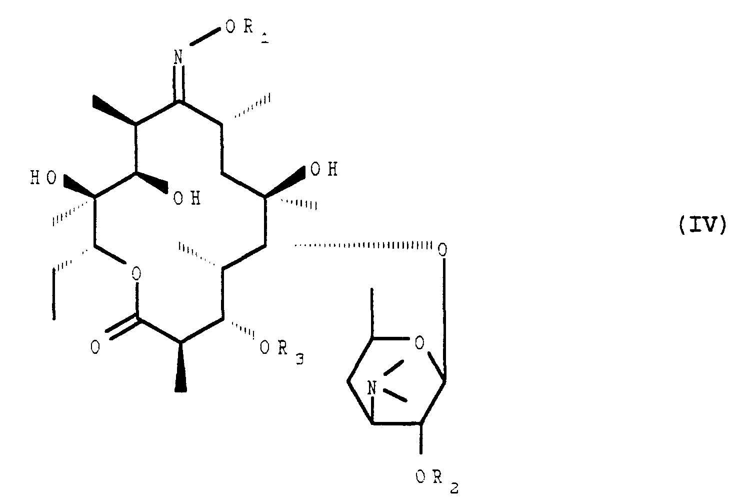 dans lequel R₁, R₂ et R₃ conservent leur signification précédente, que l'on soumet à l'action d'un agent de méthylation de l'hydroxyle en 6, pour obtenir le composé de formule (I) correspondant.

Le composé de formule (II) utilisé comme produit de départ est un produit connu décrit par Le Mahieu et Coll. dans J. Med. Chem. 17 (9) 953-956 (1974).

Dans un mode de réalisation préféré du procédé de l'invention :
- l'oxime en 9 est protégée sous forme de cétal, de thiocétal,
- les groupements 3-OH et 2'-OH sont bloqués par des groupements triméthylsilyle,
- la méthylation est réalisée au moyen de l'iodure de méthyle en présence d'une base par exemple la potasse, la soude, un hydrure tel que l'hydrure de sodium, un terbutylate de métal alcalin comme par exemple le terbutylate de potassium ou encore en présence de 1,5-diazabicyclo [4,3,0] non-5-ène ou de 1,8-diazabicyclo [5,4,0] undec-7-ène.

L'invention a également pour objet à titre de produits chimiques nouveaux les produits de formule (III) et de formule (IV) obtenus lors de la mise en oeuvre du procédé de l'invention. L'invention a plus particulièrement pour objet les produits de formules (III) et (IV) dont la préparation est donnée ci-après dans la partie expérimentale.

L'invention a également pour objet l'application des composés de formule (I), caractérisée en ce que l'on soumet le composé de formule (I) aux étapes suivantes :
- libération de l'oxime en 9,
- libération de l'hydroxyle en 3 et 2',
- protection de l'hydroxyle en 2'.

L'invention a notamment pour objet l'application caractérisée en ce que l'on soumet un composé de formule (I) à l'action de l'acide formique en présence de bisulfite de sodium ou de métabisulfite de sodium pour obtenir directement le composé de formule (V) : 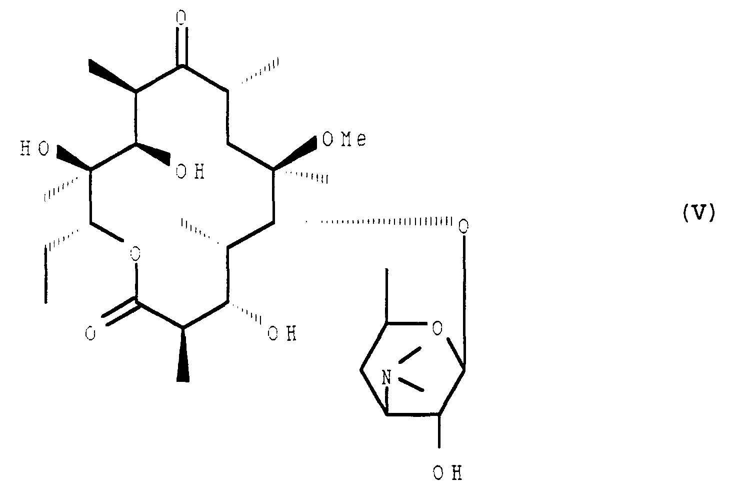 que l'on soumet à l'action d'un agent de protection de l'hydroxyle en 2' pour obtenir le composé de formule (VI) : 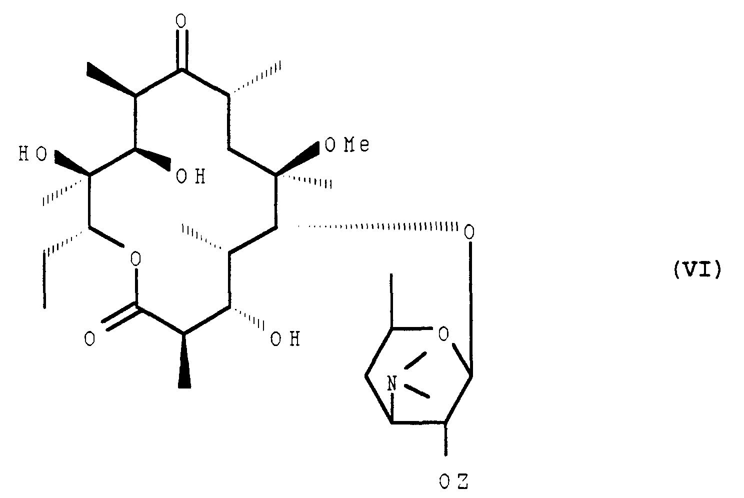 dans laquelle Z représente un groupement protecteur comme le reste d'un acide carboxylique renfermant jusqu'à 8 atomes de carbone, ou un radical trialkylsilyle, terbutyle ou triphénylméthyle.

L'invention a en outre pour objet, l'application caractérisée en ce que l'on soumet un composé de formule (I), à l'action d'un agent de libération de l'hydroxyle en 3 et en 2' pour obtenir le composé de formule (VII) : 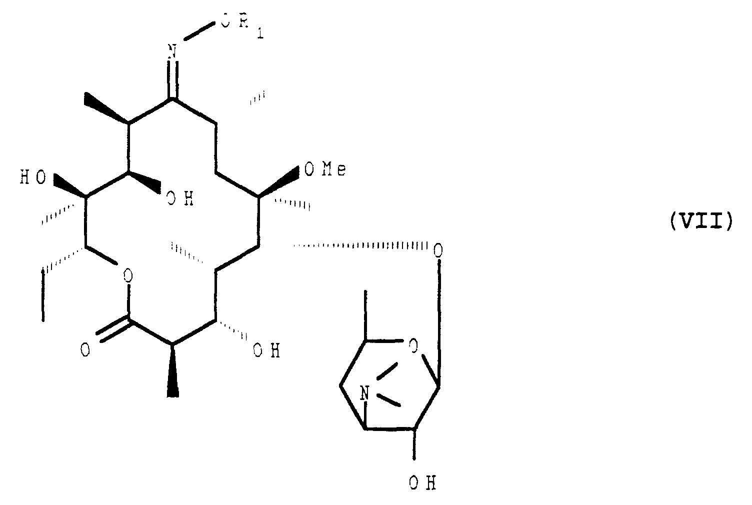 dans laquelle R₁ conserve sa signification précédente, que l'on soumet à l'action d'un agent de protection du groupement OH en 2' pour obtenir le composé de formule (VIII) : 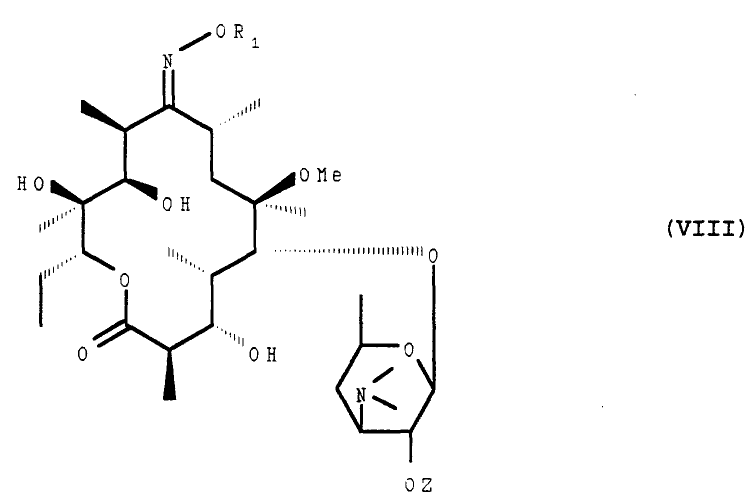 dans laquelle R₁ conserve sa signification précédente et Z représente un groupement protecteur comme défini précédemment, que l'on soumet à l'action d'un agent de libération du groupement 9-oxo pour obtenir le composé de formule (VI) correspondant : 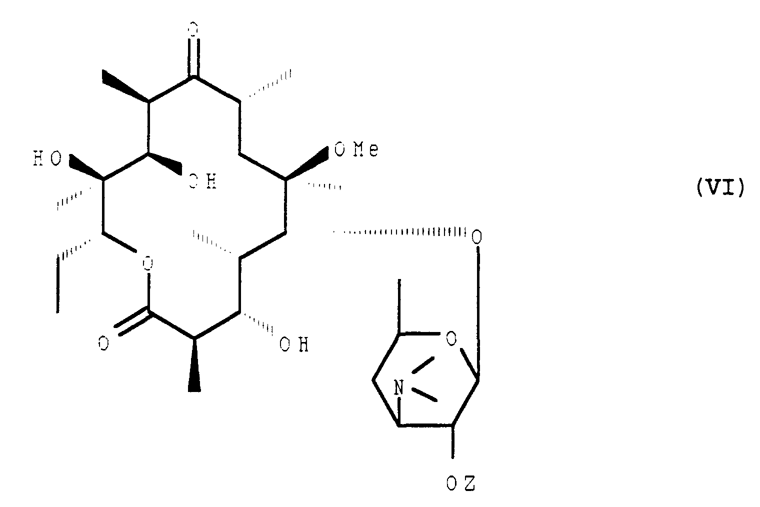 dans laquelle Z conserve sa signification précédente.

Les exemples suivants illustrent l'invention sans toutefois la limiter.

### EXEMPLE 1 : 9-O-(1-méthoxy-1-méthyléthyl) oxime de 3-O-de(2,6-didéoxy-3-C-méthyl-3-O-méthyl-alpha-L-ribo-hexopyranosyl)-2',3-O-bis(triméthylsilyl) 6-O-méthyl érythromycine,

### Stade A : 9-O-(1-méthoxy-1-méthyléthyl) oxime de 3-O-de(2,6-didéoxy-3-C-méthyl-3-O-méthyl-alpha-L-ribo-hexopyranosyl) érythromycine,

On agite pendant une demi-heure à la température ambiante 8,14 g de 9-oxime de 3-O-de(2,6-didéoxy-3-C-méthyl-3-O-méthyl-alpha-L-ribo-hexopyranosyl) érythromycine, 81,5 ml de chlorure de méthylène, 9,65 ml de 2-méthoxy propène et 2,44 g de chlorhydrate de pyridinium à 98 %. On ajoute 80 ml d'une solution saturée de NaHCO₃, agite 3 minutes. On décante la phase organique qu'on lave par 50 ml d'eau salée. On réextrait les phases aqueuses par 50 ml de CH₂Cl₂. On sèche la phase organique sur Na₂SO₄ et évapore le solvant sous pression réduite. On récupère 9 g de produit recherché. Rendement 98,5 %.

### Résultats analytiques :

RMN (CDCl₃, 300 Mhz)
0,84 (t) : CH₃-CH₂ ; 1,07 (d)-1,09 (d)-1,23 (d)-1,26 (d)x2 : les CH₃-CH ; 2,25 (s) : N(Me)₂ ; 2,48 (m) : H'₃ ; 2,64 (dq) : H₂ ; 2,72 (ql) : H₁₀ ; 3,22 (s) : OMe ; ∼3,25 : H'₂ ; 3,51 (d) : H₅ ; 3,58 (dl) : H₃ ; 3,68 (sl) : H₁₁ ; ~3,50 (m) : H'₅ ; ∼3,62 (m) : H₈ ---> E ; 4,41 (d) : H'₁ ; 5,23 (dd) : H₁₃ ; 2,36-4,48-3,58 : H mobiles.

### Stade B : 9-O-(1-méthoxy-1-méthyléthyl) oxime de 3-O-de(2,6-didéoxy-3-C-méthyl-3-O-méthyl-alpha-L-ribo-hexopyranosyl)-2',3-O-bis(triméthylsilyl) érythromycine,

On agite un mélange de 6,62 g de produit préparé au stade précédent, 66 ml de CH₂Cl₂, 2,95 ml de N-triméthylsilyl imidazole, 1,7 ml de chlorure de triméthylsilyle, 45 minutes à température ambiante. On ajoute 50 ml d'une solution saturée de NaHCO₃. On décante la phase organique qu'on lave avec 30 ml d'eau salée. On réextrait les phases aqueuses par 40 ml de CH₂Cl₂. On sèche la phase organique sur Na₂SO₄ et évapore le solvant sous pression réduite. On récupère 7,5 g de produit recherché. Rendement : 92,9 %.

### Résultats analytiques :

RMN (CDCl₃, 300 Mhz)
0,12-0,16 les OTMS ; 0,84 (t) : CH₃-CH₂ ; 1,16 (x2)-1,38-1,45-1,47-1,00-1,25 : les CH₃-CH ; 2,23 (s) : N(Me)₂ ; 2,47 (m) : H'₃ ; 2,71 (m) : H₂ et H₁₀ ; 3,16 (dd) : H'₂ ; 3,22 (s) : OMe ; 3,45 (m) : H'₅ ; 3,58 (d) : H₅ ; 3,66 : H₈ ---> E ; 3,66 (s) : H₁₁ ; 3,98 (dl) : H₃ ; 4,2 (dd) : H'₁ ; 5,14 (dd) : H₁₃ ; 1,90 (s)-3,10-4,44 : OH.

### Stade C : 9-O-(1-méthoxy-1-méthyléthyl) oxime de 3-O-de(2,6-didéoxy-3-C-méthyl-3-O-méthyl-alpha-L-ribo-hexopyranosyl)-2',3-O-bis(triméthylsilyl) 6-O-méthyl érythromycine,

On agite 1,24 g de produit préparé au stade précédent, 8,7 ml d'un mélange diméthyl sulfoxyde/tétrahydrofuranne 1/1, 190 µl de iodure de méthyle, 161 mg de potasse en poudre à 90 %, 2 heures à température ambiante. On ajoute 10 ml d'AcOEt et 10 ml d'une solution de phosphate monosodique 0,5 M. Après décantation et réextraction à l'AcOEt, on lave la phase organique par 5 ml d'eau, la sèche sur Na₂SO₄ et concentre le filtrat sous pression réduite. On obtient 1,2 g du produit recherché. Rendement : 95 %.

### Résultats analytiques :

RMN (CDCl₃, 300 Mhz)
Structure possible, on localise à 0,11 et 0,20 les SiMe₃ 0,84 (t) : CH₃-CH₂ ; 0,95 (d)-0,97(d)-1,14(d)-1,17(d) x 2 : les CH₃-CH ; 1,18-1,35-1,40-1,48 les CH₃-CH ; 2,22 (s) : N(Me)₂ ; 2,46 (m) : H'₃ ; 2,61 (ql) : H₁₀ ; 2,72 (dq) : H₂ ; 3,01 (s) : OMe ; 3,13 (dd) : H'₅ ; 3,22 (s) : OMe chaîne ; 3,45 (m) : H'₅ ; ∼3,70 : H₈ ---> E ; ∼3,68 (m) : 2H (H₃,H₅) ; 3,79 (sl) 1H ---> H₁₁ ; 4,24 (d) : H'₁ ; 5,15 (dd) : H₁₃ ; 3,29 (s) et 4,52 les OH.

### Application 1 : 2'-O-acétyl 3-O-dé(2,6-didéoxy 3-C-méthyl 3-O-méthyl alpha-L-ribo-hexopyranosyl) 6-O-méthyl érythromycine

### Stade A : 3-O-dé(2,6-didéoxy 3-C-méthyl 3-O-méthyl alpha-L-ribo-hexopyranosyl) 6-O-méthyl érythromycine

On agite un mélange de 513 mg du produit de l'exemple 1, 5 ml d'EtOH/eau 1/1, 425 mg de bisulfite de sodium, 115 µl d'acide formique, une demi-heure au reflux. Après refroidissement à température ambiante, on ajoute 5 ml d'une solution saturée de NaHCO₃. On agite le mélange 5 minutes puis on extrait par 2 fois à l'AcOEt. On lave les phases d'extraction par 5 ml d'une solution saturée de NaCl. On sèche la phase organique sur Na₂SO₄ et évapore le solvant sous vide. On obtient 180 mg de produit recherché, après chromatographie sur silice avec éluant : AcOEt 95/MeOH 3/TEA 2. Rendement 48 %.

### Résultats analytiques :

RMN (CDCl₃, 250 Mhz)
Spectre identique aux données de la littérature
5,17 (d) : H₁₃ ; 4,38 (d) : H'₁ ; 3,93 (sl) : H mobile ; 3,85 (s) : H₁₁ ; 3,68 (s) : H₅ ; 3,54 à 3,62 (m) : H₃, H'₅ : 3,24 (m) : H'₂ ; 2,98 (s) : OMe ; 2,25 (s) : N(Me)₂ ; 1,37-1,31-1,27-1,25-1,21-1,18-1,14-1,11 : les CH₃-CH ; 0,83 (t) : CH₃-CH₂.

### Stade B : 2'-O-acétyl 3-O-dé(2,6-didéoxy 3-C-méthyl 3-O-méthyl alpha-L-ribo-hexopyranosyl) 6-O-méthyl érythromycine

On soumet le produit du stade précédent à l'action de l'anhydride acétique et obtient le produit recherché.

### Application 2 : 2'-O-acétyl 3-O-dé(2,6-didéoxy 3-C-méthyl 3-O-méthyl alpha-L-ribo-hexopyranosyl) 6-O-méthyl érythromycine

### Stade A : 9-O-(1-méthoxy 1-méthyléthyl) oxime de 3-O-dé(2,6-didéoxy 3-C-méthyl 3-O-méthyl alpha-L-ribo-hexopyranosyl) 6-O-méthyl érythromycine

On ajoute à un mélange de 2,75 g du produit de l'exemple 1, 5,5 ml de tétrahydrofuranne rapidement à température ambiante, 8,25 ml de fluorure de tétrabutyl ammonium 1M dans le tétrahydrofuranne, puis on agite 45 minutes. On ajoute alors un mélange de 15 ml d'acétate d'éthyle et 15 ml d'eau glacée. Après décantation, on réextrait la phase organique par 3 ml d'eau. On ajoute à la phase aqueuse 0,82 ml d'ammoniaque concentrée. On extrait la phase aqueuse avec de l'acétate d'éthyle. On lave la phase organique par 3 ml d'une solution d'eau saturée de chlorure de sodium puis la sèche sur sulfate de sodium et évapore le solvant sous pression réduite. On récupère 2,17 g de produit recherché. Rendement 95,7 %.

### Résultats analytiques :

RMN (CDCl₃, 300 Mhz)
0,84 (t) : CH₃-CH₂ ; 0,97 (d)-1,10(d)-1,18(d)-1,24(d)-1,26(d) les CH₃-CH ; 1,20-1,40(x 2)-1,48 les CH₃-C ; 2,26 (s) : N(Me)₂ ; 2,13 (ql) : H₄ ; 2,48 (m) : H'₃ ; ∼2,66 : H₁₀ et H₂ ; 2,98 (s) : OMe en 6 ; 3,22 (s) : OMe chaîne ; ∼3,26 : H'₂ ; ∼3,54 : H₃ et H'₅ ; 3,68 (s)-3,83 (d) : H₅ et H₁₁ ; ~3,73 (m) H₈ ---> E ; 4,38 (d) : H'₁ ; 5,23 (dd) : H₁₃.

### Stade B : 9-O-(1-méthoxy 1-méthyléthyl) oxime de 2'-O-acétyl 3-O-dé(2,6-didéoxy 3-C-méthyl 3-O-méthyl alpha-L-ribo-hexopyranosyl) 6-O-méthyl érythromycine

Un mélange de 2,17 g de produit préparé au stade précédent, 22 ml de CH₂Cl₂, 390 µl d'anhydride acétique, est agité une heure et demie à la température ambiante. On ajoute 22 ml d'une solution saturée de bicarbonate de sodium. On lave la phase organique par 10 ml d'eau salée. On réextrait les phases aqueuses par CH₂Cl₂. On sèche la phase organique sur sulfate de sodium, puis évapore le solvant sous pression réduite. Le résidu obtenu est repris dans 4,25 ml d'éther isopropylique puis 14,9 ml d'heptane. Après 5 minutes d'agitation le précipité est essoré puis lavé à l'heptane. Après séchage on récupère 1,72 g de produit recherché (cristaux incolores) PF = 200°C. Rendement 74,7 %.

### Résultats analytiques :

RMN (CDCl₃, 300 Mhz)
0,83 (t) : CH₃-CH₂ ; 0,92 (d)-0,97(d)-1,17(d)-1,28(d)-1,30(d) les CH₃-CH ; 1,18-1,29-1,40-1,47 les CH₃-C ; 2,06 (s) : OAc ; 2,26 (s) : N(Me)₂ ; 2,59 (ql) : H₁₀ ; 2,69 (m) : H'₃ et H₂ ; 2,95 (s) : OMe en 6 ; 3,22 (s) : OMe chaîne ; ∼3,47 : H₃ ; H₈ et H'₅ ; 3,73 (d) : H₅ et 3,79 (sl) : H₁₁ ; 4,60 (d) : H'₁ ; 4,77 (dd) : H'₂ ; 5,23 (dd) : H₁₃ ; 1,72 (d)-3,32-4,63 : H mobiles.

### Stade C : 2'-O-acétyl 3-O-dé(2,6-didéoxy 3-C-méthyl 3-O-méthyl alpha-L-ribo-hexopyranosyl) 6-O-méthyl érythromycine

Un mélange de 180 mg de produit préparé au stade précédent, 1,8 ml de éthanol/eau 1/1, 23 µl d'acide formique à 98 %, 180 mg de bisulfite de sodium, est agité 3 heures et demie au reflux. On refroidit à la température ambiante et l'on ajoute 1,8 ml d'une solution saturée de bicarbonate de sodium. Après 3 minutes d'agitation, on extrait par 2 fois avec du CH₂Cl₂. On lave la phase organique par 2 ml d'une solution aqueuse saturée de NaCl. On sèche la phase organique sur sulfate de sodium et évapore le solvant sous pression réduite. Après purification du résidu par chromatographie sur silice en éluant avec de l'acétate d'éthyle à 2 % de tétrahydrofuranne, on récupère 43 mg de produit recherché. Rendement 27 %.

### Résultats analytiques :

| IR : | |
|---|---|
| -OH | ~3626 cm⁻¹ (Max) |
| | 3500 cm⁻¹ |
| >=O | 1735 cm⁻¹ |
| | 1689 cm⁻¹. |

## Revendications

1. Les composés de formule (I) : 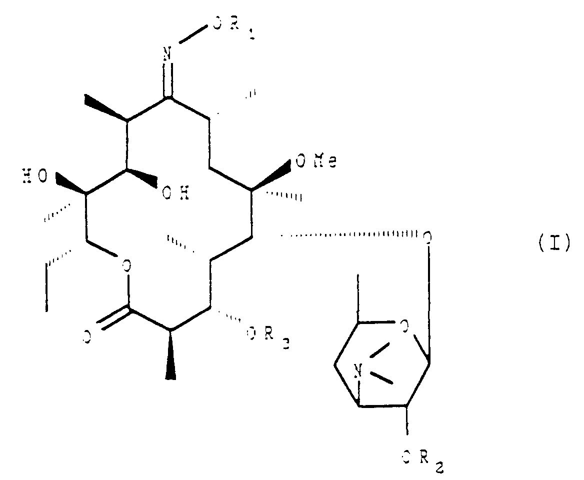 dans lesquels :
ou bien R₁ représente un radical alkyle renfermant jusqu'à 8 atomes de carbone substitué par un ou plusieurs radicaux alkyle renfermant jusqu'à 8 atomes de carbone, ou par un ou plusieurs radicaux aryle renfermant jusqu'à 14 atomes de carbone,
ou bien R₁ représente un radical aryle renfermant jusqu'à 14 atomes de carbone, éventuellement substitué par un ou plusieurs radicaux alkyle, alkényle ou alkynyle renfermant jusqu'à 8 atomes de carbone, alkoxy ou alkylthio renfermant jusqu'à 8 atomes de carbone, nitro, CF₃ ou par un ou plusieurs atomes d'halogène,
ou bien R₁ représente un radical : 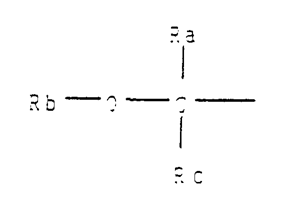 dans lequel Ra représente un radical alkyle ou alkoxy renfermant jusqu'à 8 atomes de carbone,
Rb représente un radical alkyle renfermant jusqu'à 8 atomes de carbone, éventuellement substitué par un hétéroatome,
Rc représente un atome d'hydrogène ou un radical alkyle renfermant jusqu'à 8 atomes de carbone,
R₂ et R₃, identiques ou différents, représentent un radical trialkylsilyle dans lequel le radical alkyle renferme jusqu'à 8 atomes de carbone,

2. Les composés de formule (I) tels que définis à la revendication 1 dans lesquels R₁ représente un radical 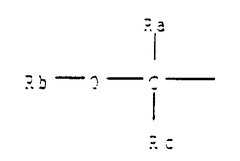 dans lequel Ra, Rb et Rc conservent la même signification que dans la revendication 1.

3. Les composés de formule (I) tels que définis à la revendication 2, dans lesquels Ra, Rb et Rc représentent un radical méthyle.

4. Les composés de formule (I) tels que définis à la revendication 1, dans lesquels R₂ et R₃ représentnt un radical triméthylsilyle.

5. Le composé de formule (I) définie à la revendication 1 dont le nom suit :
9-O-(1-méthoxy-1-méthyléthyl) oxime de 3-0-de(2,6-didéoxy-3-C-méthyl-3-O-méthyl-alpha-L-ribo-hexopyranosyl)-2'O,3-O-bis-(triméthylsilyl)-6-O-méthyl érythromycine.

6. Procédé de préparation des composés de formule (I) tels que définis à l'une quelconque des revendications 1 à 6, **caractérisé en ce que** l'on soumet le composé de formule (II) : 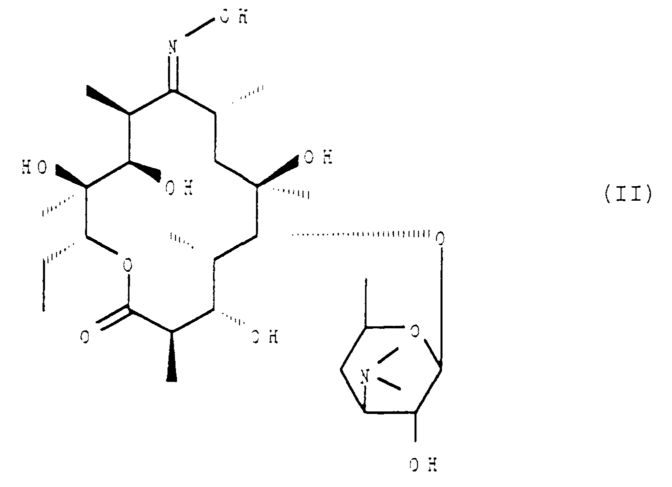 à l'action d'un agent de blocage de l'oxime en 9, pour obtenir un composé de formule (III) : 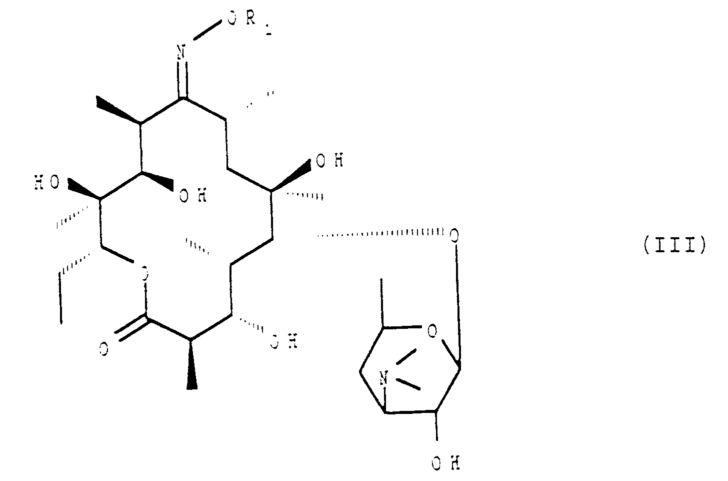 dans lequel R₁ conserve sa signification précédente, que l'on soumet à l'action d'un agent de blocage de l'hydroxyle en 3 et en 2' pour obtenir le composé de formule (IV) : 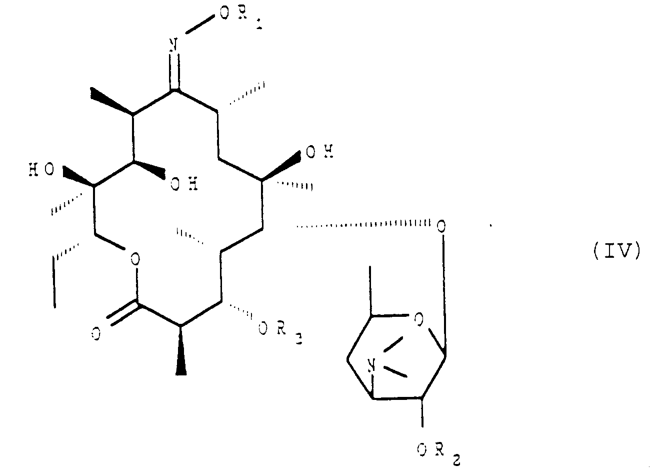 dans lequel R₁, R₂ et R₃ conservent leur signification précédente, que l'on soumet à l'action d'un agent de méthylation de l'hydroxyle en 6, pour obtenir le composé de formule (I) correspondant.

7. Procédé de préparation selon la revendication 6, **caractérisé en ce que** la méthylation du composé de formule (IV) est réalisée au moyen de l'iodure de méthyle en présence d'une base.

8. A titre de produits chimiques, les composés de formules (III) et (IV) tels que définis à la revendication 6.

9. A titre de produits chimiques définis à la revendication 8, les produits suivants :
la 9-O-(1-méthoxy-1-méthyléthyl) oxime de 3-0-de(2,6-didéoxy-3-C-méthyl-3-O-méthyl-alpha-L-ribo-hexopyranosyl) érythromycine,
la 9-O-(1-méthoxy-1-méthyléthyl) oxime de 3-0-de(2,6-didéoxy-3-C-méthyl-3-O-méthyl-alpha-L-ribo-hexopyranosyl)-2'O,3-O-bis(triméthylsilyl) érythromycine.

10. Application des composés de formule (I) tels que définis à l'une quelconque des revendications 1 à 4, **caractérisée en ce que** l'on soumet le composé de formule (I) aux étapes suivantes :
- libération de l'oxime en 9,
- libération de l'hydroxyle en 3 et 2',
- protection de l'hydroxyle en 2'.

11. Application selon la revendication 10, **caractérisée en ce que** l'on soumet un composé de formule (I) à l'action de l'acide formique en présence de bisulfite de sodium ou de métabisulfite de sodium, pour obtenir directement le composé de formule (V) : 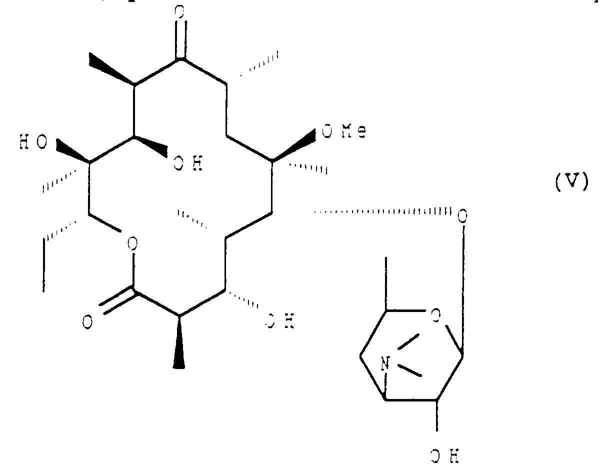 que l'on soumet à l'action d'un agent de protection de l'hydroxyle en 2' pour obtenir le composé de formule (VI) : 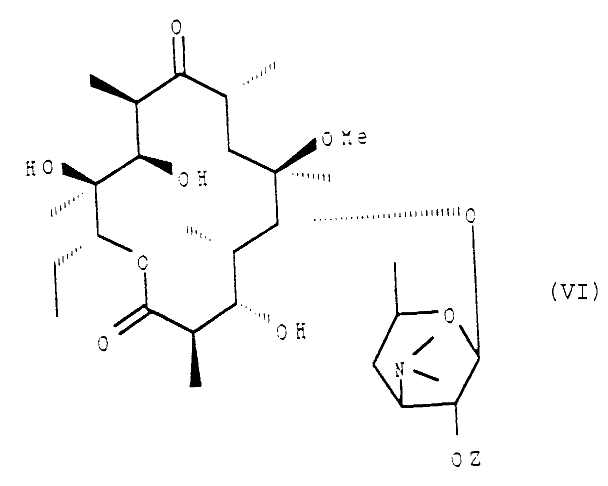 dans laquelle Z représente un groupement protecteur comme le reste d'un acide carboxylique renfermant jusqu'à 8 atomes de carbone, un radical trialkylsilyle, terbutyle ou triphénylméthyle.

12. Application selon la revendication 11, **caractérisée en ce que** l'on soumet un composé de formule (I) à l'action d'un agent de libération de l'hydroxyle en 3 et en 2' pour obtenir le composé de formule (VII) : 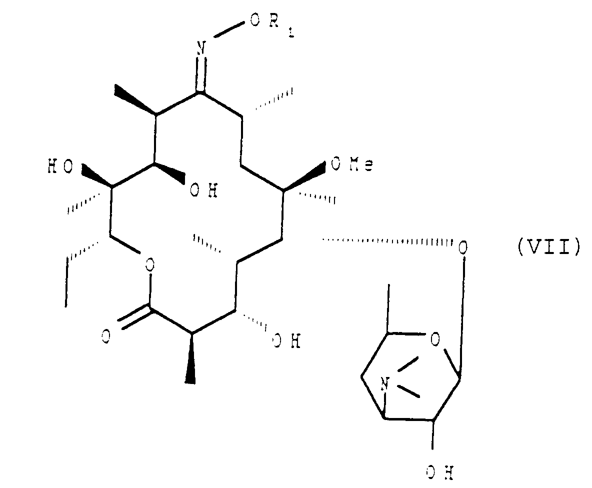 dans laquelle R₁ conserve sa signification précédente, que l'on soumet à l'action d'un agent de protection du groupement OH en 2' pour obtenir le composé de formule (VIII) : 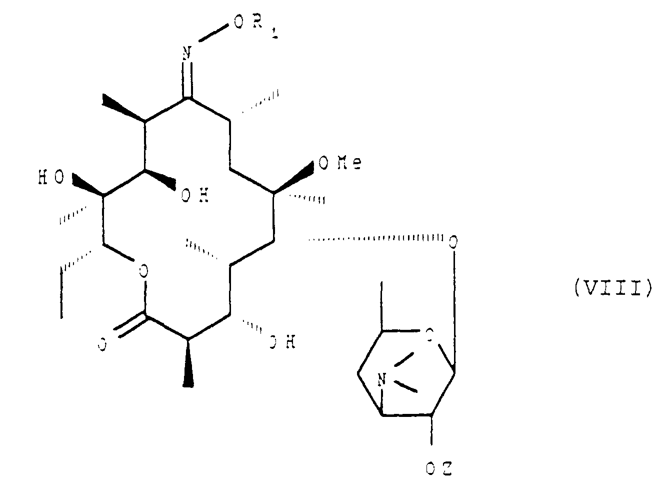 dans laquelle R₁ conserve sa signification précédente et Z représente un groupement protecteur, que l'on soumet à l'action d'un agent de libération du groupement 9-oxo pour obtenir le composé de formule (VI) correspondant : 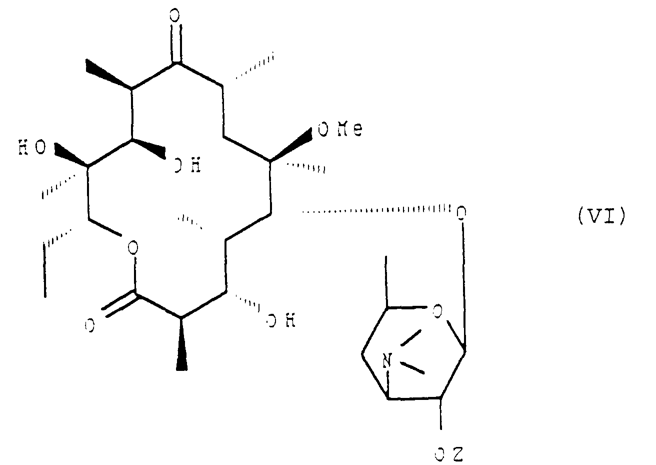 dans laquelle Z conserve sa signification précédente.

## Claims

1. The compounds of formula (I): 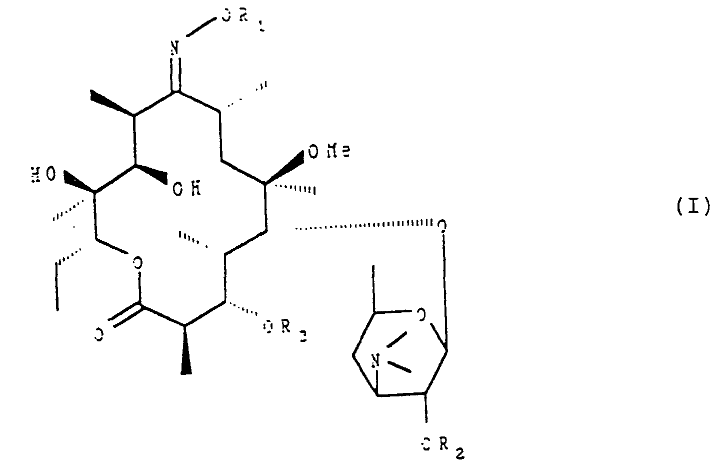 in which:
either R₁ represents an alkyl radical containing up to 8 carbon atoms substituted by one or more alkyl radicals containing up to 8 carbon atoms, or by one or more aryl radicals containing up to 14 carbon atoms,
or else R₁ represents an aryl radical containing up to 14 carbon atoms, optionally substituted by one or more alkyl, alkenyl or alkynyl radicals containing up to 8 carbon atoms, alkoxy or alkythio containing up to 8 carbon atoms, nitro, CF₃ or by one or more halogen atoms.
or R₁ represents a: 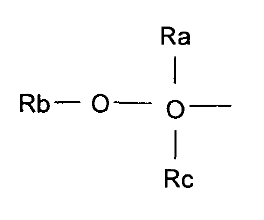 radical,
in which Ra represents an alkyl or alkoxy radical containing up to 8 carbon atoms,
Rb represents an alkyl radical containing up to 8 carbon atoms. optionally substituted by a heteroatom,
Rc represents a hydrogen atom or an alkyl radical containing up to 8 carbon atoms,
R₂ and R₃, identical or different, represent a trialkylsilyl radical in which the alkyl radical contains up to 8 carbon atoms.

2. The compounds of formula (I) as defined in claim 1 in which R₁ represents a 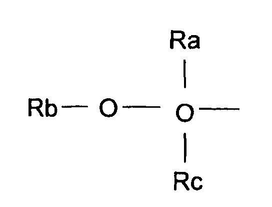 radical
in which Ra, Rb and Rc retain the same meaning as in claim 1.

3. The compounds of formula (I) as defined in claim 2, in which Ra, Rb and Rc represent a methyl radical.

4. The compounds of formula (I) as defined in claim 1, in which R₂ and R₃ represent a trimethylsilyl radical.

5. The compound of formula (I) defined in claim 1 the name of which follows:
9-0-(1-methoxy-1-methylethyl) oxime of 3-0-de(2,6-dideoxy-3-C-methyl-3-O-methyl-alpha-L-ribo-hexopyranosyl)-2'O,3-O-bis-(trimethylsilyl)-6-O-methyl erythromycin.

6. Preparation process for compounds of formula (I) as defined in one of claims 1 to 6, **characterized in that** the compound of formula (II): 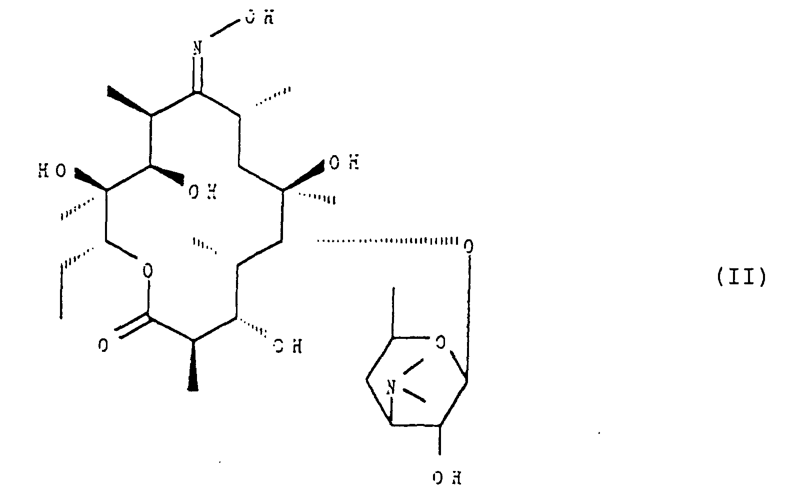 is subjected to the action of a blocking agent of the oxime in position 9, in order to obtain a compound of formula (III) : 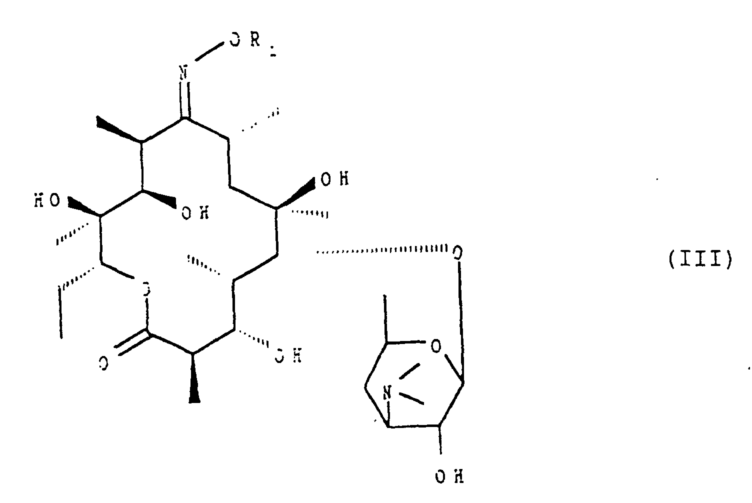 in which R₁ retains its previous meaning, which is subjected to the action of a blocking agent of the hydroxyl in position 3 and in position 2' in order to obtain the compound of formula (IV): 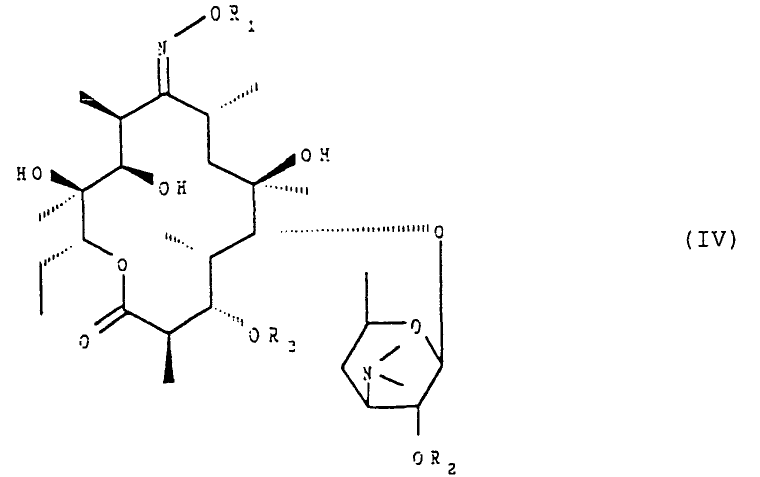 in which R₁, R₂ and R₃ retain their previous meaning, which is subjected to the action of an methylation agent of the hydroxyl in position 6, in order to obtain the corresponding compound of formula (I).

7. Preparation process according to claim 6, **characterized in that** the methylation of the compound of formula (IV) is carried out using methyl iodide in the presence of a base.

8. As chemical products, the compounds of formulae (III) and (IV) as defined in claim 6.

9. As chemical products defined in claim 8, the following products:
9-0-(1-methoxy-1-methylethyl) oxime of 3-0-de(2,6-dideoxy-3-C-methyl-3-0-methyl-alpha-L-ribo-hexopyranosyl) erythromycin,
9-O-(1-methoxy-1-methylethyl) oxime of 3-0-de(2,6-dideoxy-3-C-methyl-3-O-methyl-alpha-L-ribo-hexopyranosyl) erythromycin.

10. Use of the compounds of formula (I) as defined in any one of claim 1 to 4, **characterized in that** the compound of formula (I) is subjected to the following stages:
- release of the oxime in position 9,
- release of the hydroxyl in position 3 and 2',
- protection of the hydroxyl in position 2'.

11. Use according to claim 10, **characterized in that** a compound of formula (I) is subjected to the action of formic acid in the presence of sodium bisulphite or sodium metabisulphite, in order to directly obtain the compound of formula (V): 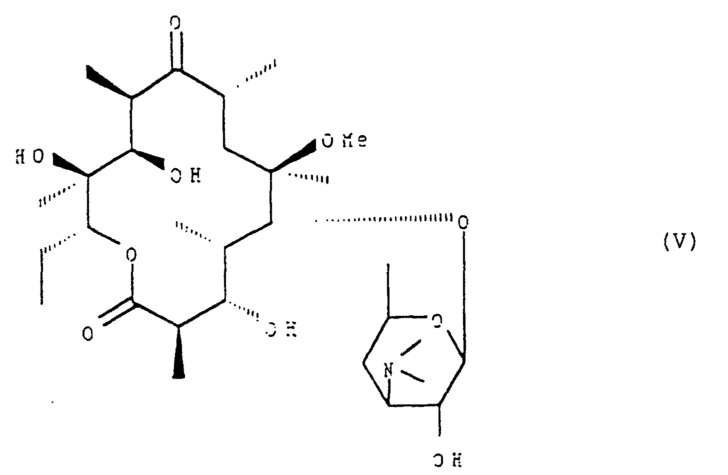 which is subjected to the action of a protection agent of the hydroxyl in position 2' in order to obtain the compound of formula (VI): 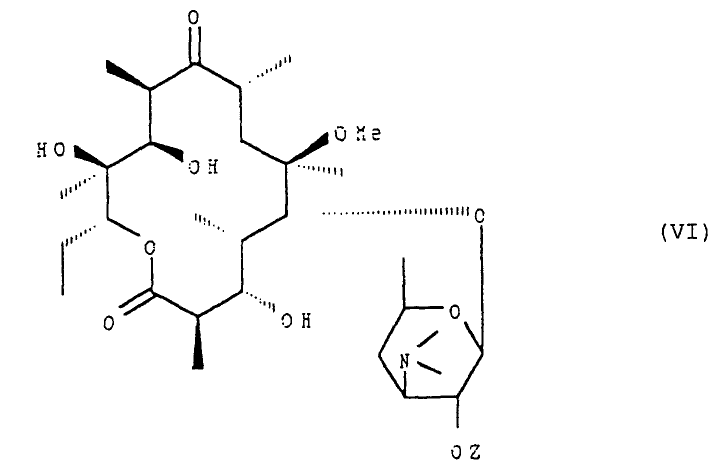 in which Z represents a protective group such as the remainder of a carboxylic acid containing up to 8 carbon atoms, a trialkylsilyl, terbutyl or triphenylmethyl radical.

12. Use according to claim 11, **characterized in that** a compound of formula (I) is subjected to the action of an agent which releases the hydroxyl in position 3 and in position 2' in order to obtain the compound of formula (VII) : 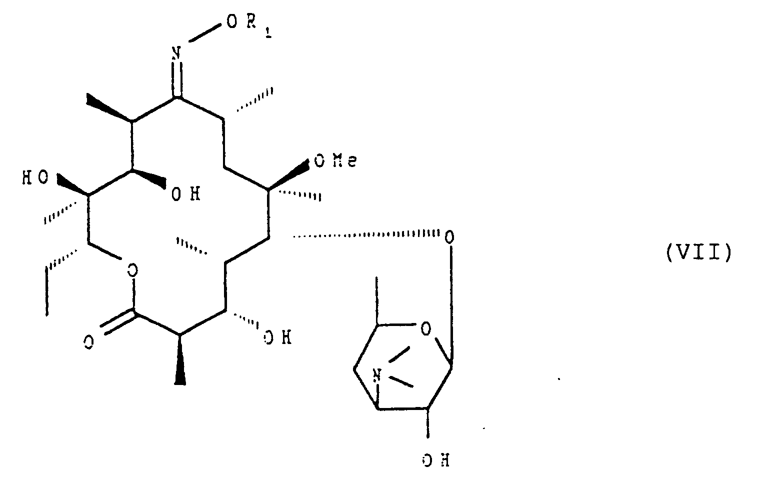 in which R₁ retains its previous meaning, which is subjected to the action of a protection agent of the OH group in position 2' in order to obtain the compound of formula (VIII): 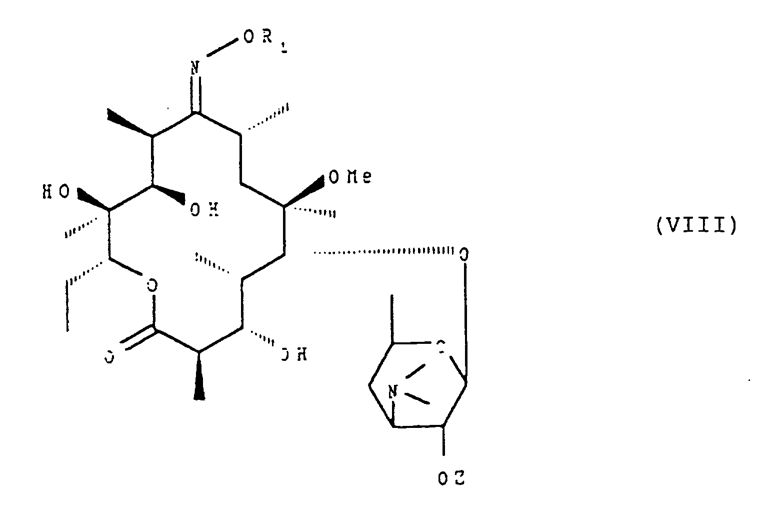 in which R₁ retains its previous meaning and Z represents a protective group, which is subjected to the action of an agent which releases the 9-oxo group in order to obtain the corresponding compound of formula (VI): 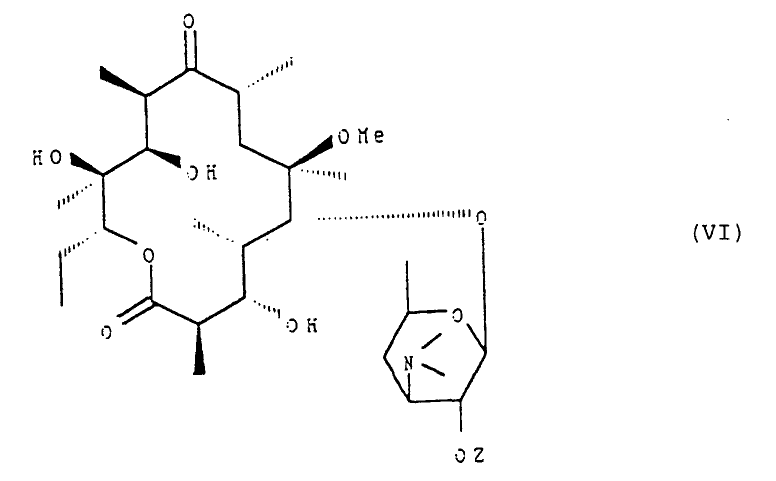 in which Z retains its previous meaning.

## Patentansprüche

1. Verbindungen der Formel (I) 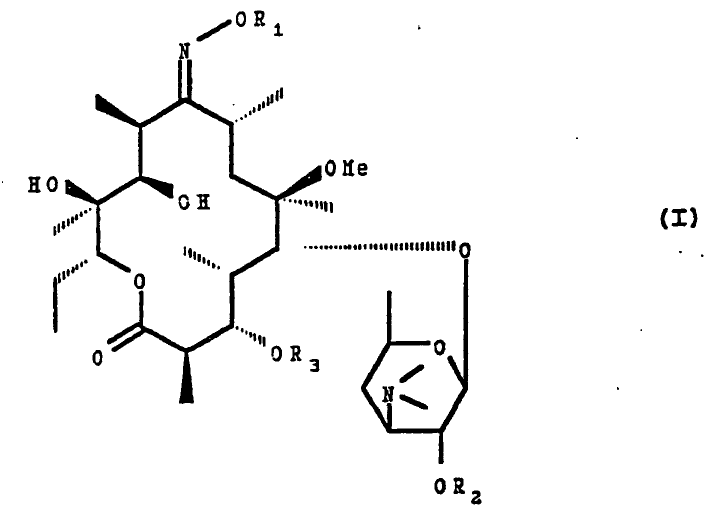 in der
entweder R₁ einen Rest Alkyl mit bis zu 8 Kohlenstoffatomen darstellt, substituiert durch einen oder mehrere Reste Alkyl mit bis zu 8 Kohlenstoffatomen oder durch einen oder mehrere Reste Aryl mit bis zu 14 Kohlenstoffatomen,
oder R₁ einen Rest Aryl mit bis zu 14 Kohlenstoffatomen darstellt, gegebenenfalls substituiert durch einen oder mehrere Reste Alkyl, Alkenyl oder Alkinyl mit bis zu 8 Kohlenstoffatomen, Alkoxy oder Alkylthio mit bis zu 8 Kohlenstoffatomen, Nitro, CF₃ oder durch ein oder mehrere Halogenatome,
oder R₁ einen Rest Ra 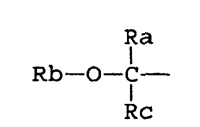 darstellt, worin Ra einen Rest Alkyl oder Alkoxy mit bis zu 8 Kohlenstoffatomen bedeutet,
Rb einen Rest Alkyl mit bis zu 8 Kohlenstoffatomen bedeutet, gegebenenfalls substituiert durch ein Heteroatom,
Rc ein Wasserstoffatom oder einen Rest Alkyl mit bis zu 8 Kohlenstoffatomen bedeutet,
R₂ und R₃, gleich oder verschieden, einen Rest Trialkylsilyl darstellen, worin der Rest Alkyl bis zu 8 Kohlenstoffatome umfaßt.

2. Verbindungen der Formel (I) wie in Anspruch 1 definiert, in der R₁ einen Rest 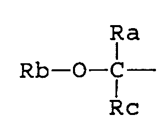 darstellt, worin Ra, Rb und Rc die gleiche Bedeutung wie in Anspruch 1 beibehalten.

3. Verbindungen der Formel (I) wie in Anspruch 2 definiert, in der Ra, Rb und Rc einen Rest Methyl darstellen.

4. Verbindungen der Formel (I) wie in Anspruch 1 definiert, in der R₂ und R₃ einen Rest Trimethylsilyl darstellen.

5. Verbindung der Formel (I) wie in Anspruch 1 definiert mit dem folgenden Namen:
9-O-(1-Methoxy-1-methylethyl)-oxim von 3-O-de-(2,6-Dideoxy-3-Cmethyl-3-O-methyl-alpha-L-ribo-hexopyranosyl)-2'O,3-O-bis-(trimethylsilyl)-6-O-methyl-erythromycin.

6. Verfahren zur Herstellung der Verbindungen der Formel (I) wie in irgendeinem der Ansprüche 1 bis 5 definiert, **dadurch gekennzeichnet, daß** man die Verbindung der Formel (II) 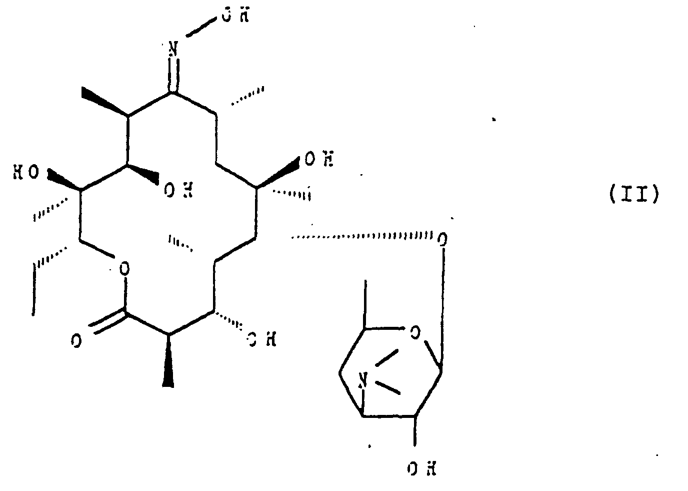 der Einwirkung eines Mittels zur Blockierung des Oxims in 9 unterzieht, um eine Verbindung der Formel (III) 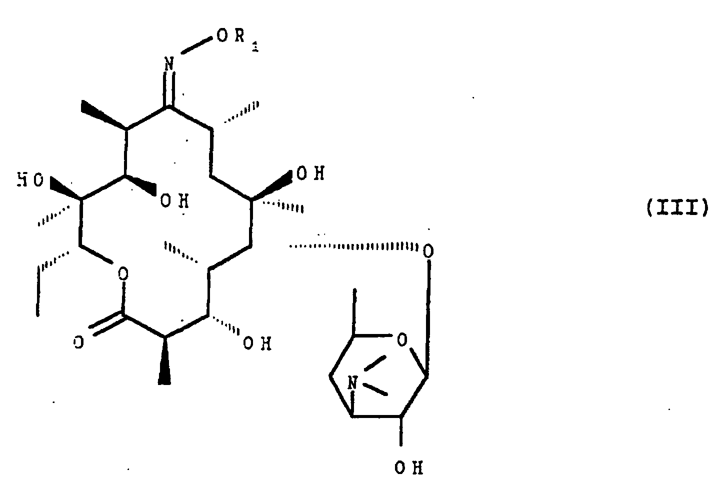 zu erhalten, in der R₁ seine vorstehende Bedeutung beibehält, die man dann der Einwirkung eines Mittels zur Blockierung des Hydroxyls in 3 und in 2' unterzieht, um die Verbindung der Formel (IV) 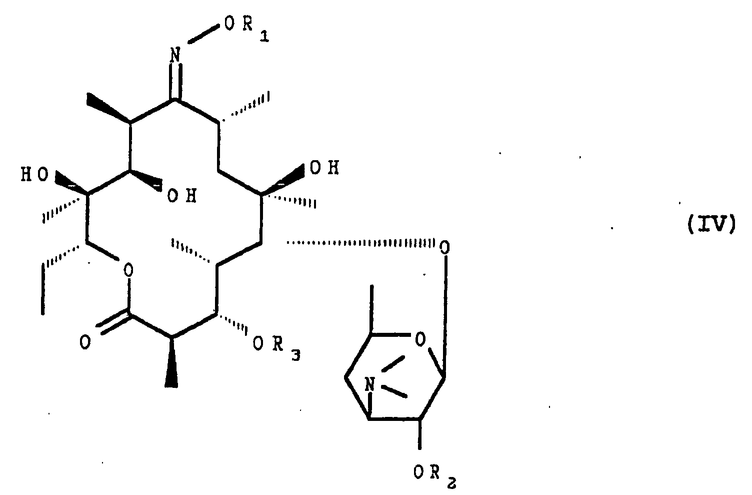 zu erhalten, in der R₁, R₂ und R₃ ihre vorstehende Bedeutung beibehalten, die man anschließend der Einwirkung eines Mittels zur Methylierung des Hydroxyls in 6 unterzieht, um die entsprechende Verbindung der Formel (I) zu erhalten.

7. Verfahren zur Herstellung nach Anspruch 6, **dadurch gekennzeichnet, daß** die Methylierung der Verbindung der Formel (IV) mit Hilfe von Methyliodid in Anwesenheit einer Base durchgeführt wird.

8. Als chemische Produkte die Verbindungen der Formeln (III) und (IV) wie in Anspruch 6 definiert.

9. Als chemische Produkte wie in Anspruch 8 definiert die folgenden Produkte:
9-O-(1-Methoxy-1-methylethyl)-oxim von 3-O-de-(2,6-Dideoxy-3-Cmethyl-3-O-methyl-alpha-L-ribo-hexopyranosyl)-erythromycin,
9-O-(1-Methoxy-1-methylethyl)-oxim von 3-O-de-(2,6-Dideoxy-3-Cmethyl-3-O-methyl-alpha-L-ribo-hexopyranosyl)-2'O,3-O-bis-(trimethylsilyl)-erythromycin.

10. Verwendung der Verbindungen der Formel (I) wie in irgendeinem der Ansprüche 1 bis 4 definiert, **dadurch gekennzeichnet, daß** man die Verbindung der Formel (I) den folgenden Stufen unterzieht:
- Freisetzung des Oxims in 9,
- Freisetzung des Hydroxyls in 3 und in 2',
- Schutz des Hydroxyls in 2'.

11. Verwendung nach Anspruch 10, **dadurch gekennzeichnet, daß** man eine Verbindung der Formel (I) der Einwirkung von Ameisensäure in Anwesenheit von Natriumbisulfit oder Natriummetabisulfit unterzieht, um direkt die Verbindung der Formel (V) 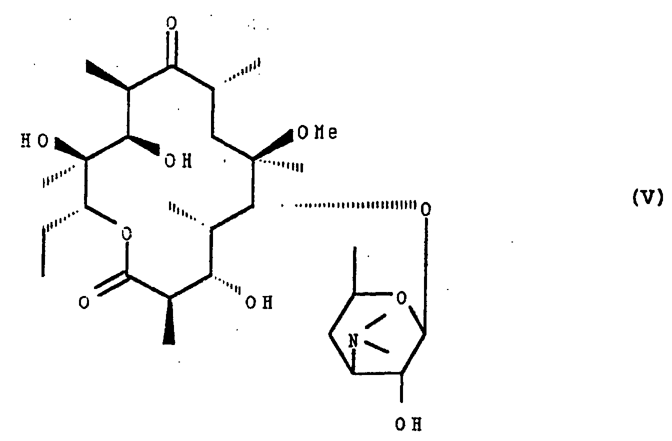 zu erhalten, die man danach der Einwirkung eines Mittels zum Schutz des Hydroxyls in 2' unterzieht, um die Verbindung der Formel (VI) 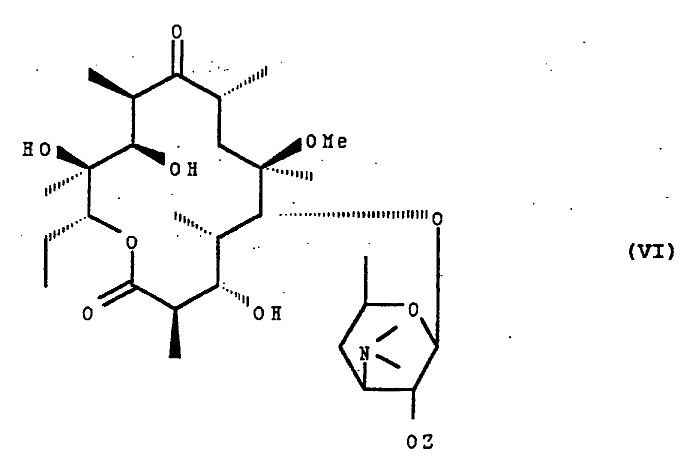 zu erhalten, in der Z eine Schutzgruppe darstellt wie den Rest einer Carbonsäure mit bis zu 8 Kohlenstoffatomen, einen Rest Trialkylsilyl, tert.-Butyl oder Triphenylmethyl.

12. Verwendung nach Anspruch 11, **dadurch gekennzeichnet, daß** man eine Verbindung der Formel (I) der Einwirkung eines Mittels zum Freisetzen des Hydroxyls in 3 und in 2' unterzieht, um die Verbindung der Formel (VII) 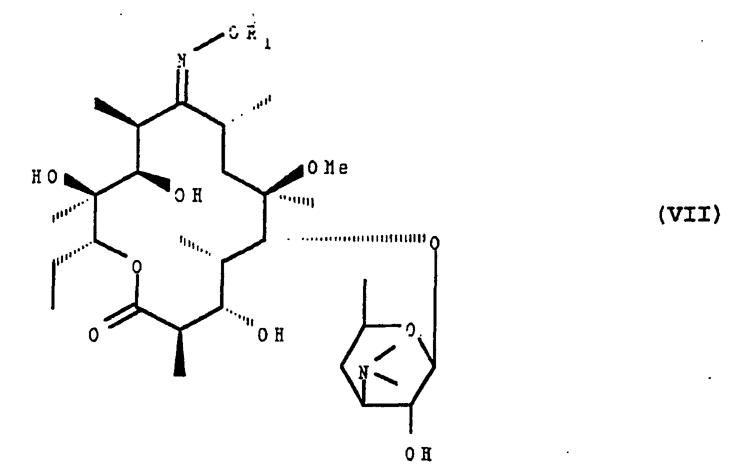 zu erhalten, in der R₁ seine vorstehende Bedeutung beibehält, die man der Einwirkung eines Mittels zum Schutz der Gruppe OH in 2' unterzieht, um die Verbindung der Formel (VIII) 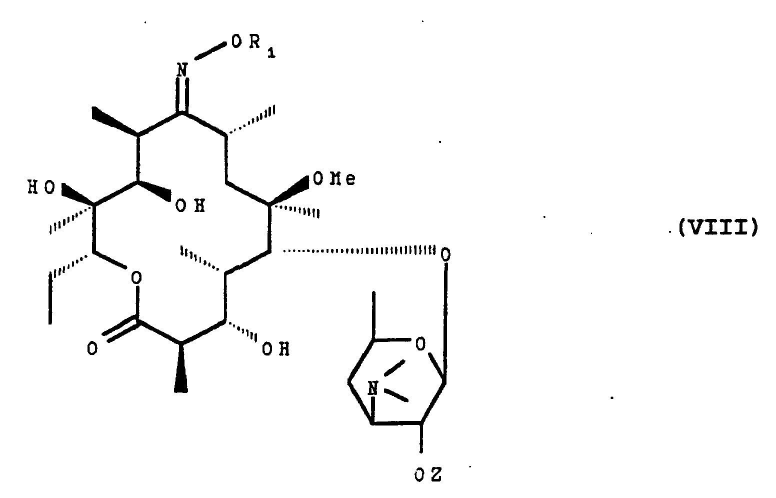 zu erhalten, in der R₁ seine vorstehende Bedeutung beibehält und Z eine Schutzgruppe darstellt, die man dann der Einwirkung eines Mittels zur Freisetzung der Gruppe 9-Oxo unterzieht, um die entsprechende Verbindung der Formel (VI) 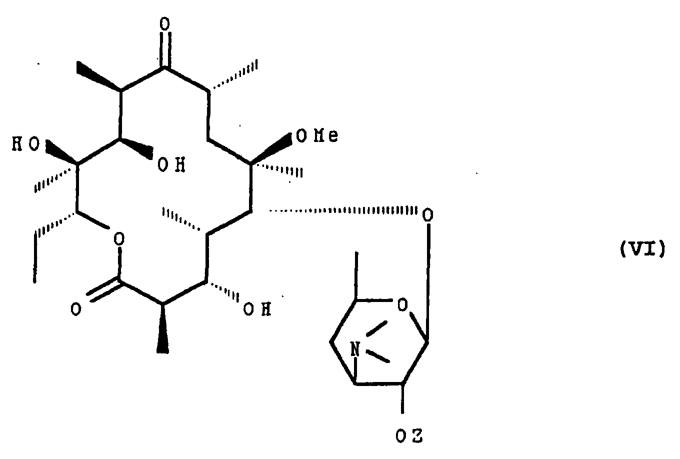 zu erhalten, in der Z seine vorstehende Bedeutung beibehält.
